# EUROPEAN PATENT APPLICATION

(11) **EP 4 748 439 A2**
(43) Date of publication of application: **27.05.2026**
(21) Application number: 26170310.2
(22) Date of filing: 31.07.2020
(51) Int. Cl.: A61P 9/12

(54) **A PEPTOID-PEPTIDE HYBRID, NMEG-ALPHA-CGRP, AND ITS USE IN CARDIOVASCULAR DISEASES**

(30) Priority: 31.07.2019 US 201962880749 P
(62) Divisional of application: 20757457.5
(71) Applicant: University of South Carolina, Columbia SC 29208 (US); Board of Trustees of the University of Arkansas, Little Rock, AR 72207 (US)
(72) Inventor: POTTS, Jay D, Columbia, 29212 (US); KUMAR, Ambrish, Columbia, 29209 (US); DIPETTE, Donald J, Blythewood, 29016 (US); SERVOSS, Shannon, Fayetteville, 72703 (US)
(74) Representative: Swindell & Pearson Limited

(57) **Abstract**

NMEG-αCGRP, a biologically active molecule, and systems and methods of use therefore, subcutaneous administration of NMEG-αCGRP, a non-toxic peptoid-peptide hybrid that possesses hypotensive action, employed as a therapeutic agent to treat and prevent various cardiovascular diseases, including, heart failure (pressure, as well as volume, overload), myocardial infarction, cardiomyopathy, and hypertension.

## Description

### BACKGROUND OF THE INVENTION

### 1) Field of the Invention

The present invention relates to NMEG-αCGRP, a biologically active molecule, and systems and methods of use therefore, subcutaneous administration of NMEG-αCGRP, a non-toxic peptoid-peptide hybrid that possesses hypotensive action, employed as a therapeutic agent to treat and prevent various cardiovascular diseases, including, heart failure (pressure, as well as volume, overload), myocardial infarction, hypertension, cardiac hypertrophy, coronary artery disease, high blood pressure, stroke, dilated cardiomyopathy, idiopathic dilated cardiomyopathy, inherited cardiomyopathy, diabetic-cardiomyopathy, cardiomyopathy induced by chemotherapy (such as doxorubicin) or toxins, cardiac ischemia, and hypertension induced heart failure and kidney damage, and cardiac remodeling induced during pregnancy, etc.

### 2) Description of Related Art

A number of diseases affecting the health of heart and blood vessels collectively fall under the category of cardiovascular diseases (CVD). Some of the examples of CVD are- coronary artery disease, heart attack, heart failure, myocardial ischemia, high blood pressure, hypertension, myocardial infarction, and stroke. CVD is the leading global cause of death of both men and women, including the United States. On the basis of 2016 mortality data, CVD currently claims more lives each year than cancer and chronic lung disease combined. Globally, more than 17 million deaths in 2016 were caused by CVD that was 14.5% more than 2006, and it is expected to rise to > 23.6 million deaths by 2030. In the United States, nearly 1 in 3 deaths is accounted by the CVD.

In 2015, approximately 41.5% of the U.S. population had at least one CVD condition (www.cdc.gov), and The American Heart Association (AHA) estimates that by 2035, 45.1% of the US population would have some form of CVD. The prevalence of CVD in adults (persons ≥20 years of age) is 48.0% and increases with age in both males and females. These numbers show that cardiac and related diseases are placing a heavy financial burden on the economy and the health care system. The total costs (direct and indirect treatment) of CVD in the USA continue to rise - in 2016 it was $555 billion and is expected to reach $1.1 trillion in 2035. Although there are several classes of drugs are available to treat and prevent cardiac diseases, the 5-year survival rate is still only 50%. Since no satisfactory cure is available for CVD, more effective therapeutic strategies are needed to be established.

Accordingly, it is an object of the present invention to provide an improved vasodilator and systems of use therefor. αCGRP is a potent vasodilator and has been shown to help in treating heart failure in rodent models. The problem with using it in human is its very short half-life in serum: it only last about 5-7 min. The inventors have designed and created a new peptide that is more stable and resistant to degradation and shows a similar biological activity to the natural peptide.

### SUMMARY OF THE INVENTION

The above objectives are accomplished according to the present invention by providing in a first embodiment a biologically active molecule. The molecule may include coupling a peptoid monomer to an end terminus of a peptide and encapsulating same in an alginate polymer. Further, the peptoid monomer may comprise N-methoxyethylglycine. Still yet, the peptoid monomer may be coupled to an N-terminus end of α-CGRP peptide. Further still, the peptoid-peptide hybrid is formulated to be administered subcutaneously. Again, the alginate polymer may comprise an unbranched polyanionic polysaccharides of 1-4 linked α-L-guluronic acid and β-D-mannuronic acid.

In an alternative embodiment, a method is provided for treating or preventing a cardiovascular disease. The method may include administering a therapeutically effective amount of a peptoid monomer coupled to an end terminus of a peptide, wherein the peptoid monomer coupled to an end terminus of a peptide may be encapsulated in an alginate polymer, and the peptoid monomer coupled to an end terminus of a peptide may be administered prior to onset or after initiation of symptoms. Further, the cardiovascular disease may be selected from the group consisting of heart failure, myocardial infarction, hypertension, cardiac hypertrophy, coronary artery disease, high blood pressure, stroke, dilated cardiomyopathy, idiopathic dilated cardiomyopathy, inherited cardiomyopathy, diabetic-cardiomyopathy, cardiomyopathy induced by chemotherapy (such as doxorubicin or toxins, cardiac ischemia, hypertension induced heart failure, or cardiac remodeling induced during pregnancy. Further yet, the peptoid monomer may be N-methoxyethylglycine. Still again, the peptoid monomer may be coupled to an N-terminus end of α-CGRP peptide. Further yet, the peptoid monomer coupled to an end terminus of a peptide may be administered subcutaneously. Yet still, the alginate polymer may comprise an unbranched polyanionic polysaccharides of 1-4 linked α-L-guluronic acid and β-D-mannuronic acid.

In a still further embodiment, a novel peptoid-peptide hybrid for use in preventing or treating cardiovascular diseases is provided. The hybrid may include an α-CGRP agonist analogue containing at least two peptoid monomers at an N-terminal end of a α-CGRP peptide. Still yet, the at least two peptoid monomers may comprise N-methoxyethylglycine. Further still, the hybrid exhibits increased stability in human plasma as compared to naturally occurring α-CGRP. Still further, the cardiovascular disease may be selected from the group consisting of heart failure, myocardial infarction, hypertension, cardiac hypertrophy, coronary artery disease, high blood pressure, stroke, dilated cardiomyopathy, idiopathic dilated cardiomyopathy, inherited cardiomyopathy, diabetic-cardiomyopathy, cardiomyopathy induced by chemotherapy (such as doxorubicin or toxins, cardiac ischemia, hypertension induced heart failure, or cardiac remodeling induced during pregnancy. Further still, the novel peptoid-peptide hybrid may be encapsulated in an alginate polymer. Still yet, the alginate polymer may comprise an unbranched polyanionic polysaccharides of 1-4 linked α-L-guluronic acid and β-D-mannuronic acid.

### BRIEF DESCRIPTION OF THE DRAWINGS

The construction designed to carry out the invention will hereinafter be described, together with other features thereof. The invention will be more readily understood from a reading of the following specification and by reference to the accompanying drawings forming a part thereof, wherein an example of the invention is shown and wherein:
Figure 1 shows the chemical structure of NMEG-αCGRP.
Figure 2 shows MALDI-TOF analysis of the synthesized peptoid-peptide hybrid NMEG-αCGRP.
Figure 3 shows a graph of NMEG-αCGRP dose response curves.
Figure 4A shows representative phase contrast images taken on day 7 after NMEG-αCGRP treatments (0.5, 1.5, and 5 µM) to cardiac H9C2 cells.
Figure 4B shows bar diagrams showing the viability of cardiac H9C2 cells after 7 days treatment with NMEG-αCGRP as determined by trypan blue cell viability assay.
Figure 4C shows representative phase contrast images of HL-1 cells taken on day 7 after NMEG-αCGRP treatment (5 µM).
Figure 4D shows live cells were quantitated by trypan blue cell viability assay and plotted as fold change.
Figure 5 shows at: (A) electrospray method used to encapsulate α-CGRP in alginate polymer; (B) prepared alginate-only and alginate-α-CGRP microcapsules were photographed; (C) measurement and plotting of (B); (D) *in vitro* α-CGRP release assay showing amount of α-CGRP released in supernatant from alginate-α-CGRP microcapsules; (E) a bar diagram showing number of live H9C2 cells, as measured by trypan-blue cell viability assay; and (F) viability of mouse HL-1 cardiac cells in presence of alginate-α-CGRP microcapsules (10 µM).
Figure 6 shows at: (A) representative echocardiograms showing short axis B- and M-mode 2D echocardiography performed after 28 days delivery of alginate-α-CGRP; and at (B) and (C) percentage fractional shortening (FS) and ejection fraction (EF) was calculated at various time points and plotted.
Figure 7 shows at: (A) representative images showing the size of the hearts after 28 days delivery of alginate-α-CGRP microcapsules; (B and C) bar diagrams showing the ratio of wet heart weight/tibia length, and wet lung weight/tibia length; (D) paraffin-embedded LV sections were stained with H&E, WGA stain; (E) stained sections were used to measure cardiomyocyte size in LVs by NIH-ImageJ software and plotted; (F) LV collagen content was quantitated by NIH-ImageJ software and plotted.
Figure 8 shows at: (A) Western blot showing level of cleaved caspase-3 protein in LVs from sham, sham-alginate-α-CGRP, TAC, and TAC-alginate-α-CGRP; (B) representative fluorescence images showing cleaved caspase-3 staining (green) to detect apoptosis in the LV sections; (C) cleaved caspase-3 positive cells (green) were counted and plotted as the mean ± SEM; (D and E) fluorescence images showing 4-HNE staining in the paraffin-embedded LV sections; and (F) bar diagrams showing glutathione (GSH) level in the LVs.

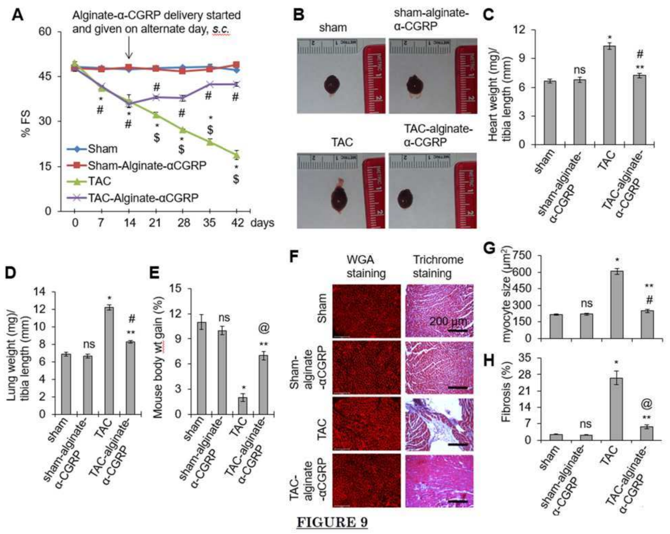

Figure 9 shows at: (A) a graph showing %FS in sham, sham-alginate-α-CGRP, TAC-only, and TAC-alginate-α-CGRP groups of mice; (B) representative images showing the size of hearts after 28 days delivery of alginate-α-CGRP microcapsules; (C) ratio of wet heart weight/tibia length was plotted as mean ± SEM; (D) a bar diagram showing ratio of wet lung weight/tibia length as mean ± SEM; (E) a bar diagram showing mice weight gain (in percentage) during the course of experiment as mean ± SEM; (F) representative histology images showing size of cardiomyocytes (WGA staining) and level of fibrosis (trichrome-collagen staining) in the LVs from different groups of mice; (G) cardiomyocyte size; and (H) percent fibrosis quantitated using NIH-ImageJ software and plotted.
Figure 10 shows a heart failure experiment protocol of the current disclosure.
Figure 11 provides a graph showing the systolic pressure, as measured by tail-cuff blood pressure method, after subcutaneous injection of various concentrations of alginate-α-CGRP microcapsules in mice.

It will be understood by those skilled in the art that one or more aspects of this invention can meet certain objectives, while one or more other aspects can meet certain other objectives. Each objective may not apply equally, in all its respects, to every aspect of this invention. As such, the preceding objects can be viewed in the alternative with respect to any one aspect of this invention. These and other objects and features of the invention will become more fully apparent when the following detailed description is read in conjunction with the accompanying figures and examples. However, it is to be understood that both the foregoing summary of the invention and the following detailed description are of a preferred embodiment and not restrictive of the invention or other alternate embodiments of the invention. In particular, while the invention is described herein with reference to a number of specific embodiments, it will be appreciated that the description is illustrative of the invention and is not constructed as limiting of the invention. Various modifications and applications may occur to those who are skilled in the art, without departing from the spirit and the scope of the invention, as described by the appended claims. Likewise, other objects, features, benefits and advantages of the present invention will be apparent from this summary and certain embodiments described below, and will be readily apparent to those skilled in the art. Such objects, features, benefits and advantages will be apparent from the above in conjunction with the accompanying examples, data, figures and all reasonable inferences to be drawn therefrom, alone or with consideration of the references incorporated herein.

### DETAILED DESCRIPTION OF A PREFERRED EMBODIMENT

With reference to the drawings, the invention will now be described in more detail. Unless defined otherwise, all technical and scientific terms used herein have the same meaning as commonly understood to one of ordinary skill in the art to which the presently disclosed subject matter belongs. Although any methods, devices, and materials similar or equivalent to those described herein can be used in the practice or testing of the presently disclosed subject matter, representative methods, devices, and materials are herein described.

Unless specifically stated, terms and phrases used in this document, and variations thereof, unless otherwise expressly stated, should be construed as open ended as opposed to limiting. Likewise, a group of items linked with the conjunction "and" should not be read as requiring that each and every one of those items be present in the grouping, but rather should be read as "and/or" unless expressly stated otherwise. Similarly, a group of items linked with the conjunction "or" should not be read as requiring mutual exclusivity among that group, but rather should also be read as "and/or" unless expressly stated otherwise.

Furthermore, although items, elements or components of the disclosure may be described or claimed in the singular, the plural is contemplated to be within the scope thereof unless limitation to the singular is explicitly stated. The presence of broadening words and phrases such as "one or more," "at least," "but not limited to" or other like phrases in some instances shall not be read to mean that the narrower case is intended or required in instances where such broadening phrases may be absent.

### ABBREVIATIONS:

4-HNE: 4-hydroxynonenal
α-CGRP or αCGRP: alpha-calcitonin gene-related peptide
A-PLO: alginate-poly-L-ornithine
BP: Blood pressure
CaCl₂: calcium chloride
CVD: cardiovascular diseases
EF: ejection fraction
FS: fractional shortening
GSH: Glutathione
KO: knock-out
LV: left ventricle
NMEG: N-methoxyethylglycine
RP-HPLC: reversed-phase high-performance liquid chromatography
S.C.: subcutaneous
TAC: transverse aortic constriction
UV: ultraviolet
WGA: Wheat germ agglutinin

Alpha-calcitonin gene related peptide (a-CGRP), a 37-amino acid regulatory neuropeptide, is one of the most potent vasodilators known. Several lines of evidences demonstrated by the inventors' laboratory suggest that α-CGRP has a cardio-protective role in various cardiovascular diseases, including hypertension, heart failure, and myocardial ischemia.

Using α-CGRP knock out mice, the inventors' laboratory demonstrated that transverse aortic constriction (TAC) induced pressure-overload significantly enhanced the cardiac hypertrophy and dysfunction, cardiac apoptosis, fibrosis and inflammation, and mortality of α-CGRP knock out mice compared to TAC wild-type mice. The inventors also demonstrated that exogenous administration of native α-CGRP protects the heart from pressure-overload induced heart failure in wild-type mice. α-CGRP significantly preserves the heart at functional and anatomical levels in pressure-overload mice. However, the short half-life of α-CGRP (~5.5 min in the human plasma) limits its use as a therapeutic agent in humans. The present disclosure is aimed at developing and testing a novel α-CGRP agonist analogue with extended stability and efficacy in human plasma. Recently, the inventors have chemically synthesized a peptoid-peptide hybrid of α-CGRP by coupling a peptoid monomer N-methoxyethylglycine (NMEG) molecule to the N-terminus end of the human α-CGRP peptide (the inventors termed this NMEG-αCGRP).

The inventors' *in vivo* data demonstrates that NMEG-αCGRP is a biologically active molecule as subcutaneous administration of NMEG-αCGRP lowers the blood pressure in wild-type mice. Synthesized NMEG-αCGRP exhibits no cellular toxicity when incubated with two different cardiac cell lines, rat H9C2 cells and mouse HL-1 cells. As NMEG-αCGRP is non-toxic and possess hypotensive action, it can be a potential therapeutic agent to treat and prevent various cardiovascular diseases, including, heart failure (pressure, as well as volume, overload), myocardial infarction, hypertension, cardiac hypertrophy, coronary artery disease, high blood pressure, stroke, dilated cardiomyopathy, idiopathic dilated cardiomyopathy, inherited cardiomyopathy, cardiomyopathy induced by chemotherapy (such as doxorubicin) or toxins, diabetic-cardiomyopathy, cardiac ischemia, and hypertension induced heart failure and kidney damage, and cardiac remodeling induced during pregnancy. The success of this technology will have the potential to dramatically change conventional drug therapies used presently to treat the failing heart.

The current disclosure provides a native peptide that is modified by another naturally occurring change to the amino acid glycine. By adding this to the peptide, it makes it much more stable and resistant to degradation. The peptoid has similar biological activity and is not toxic to two cardiac myocyte cell lines *in vitro.* The chemistry is not difficult to use and the peptide is easy to handle.

Peptide α-CGRP is the most potent vasodilator known and exhibits positive chronotropic and inotropic effects. Systemic administration of α-CGRP decreases blood pressure in normotensive and hypertensive animals and humans. Various animal and cell culture based studies confirm that α-CGRP decreases angiotensin II activity, increases cardiac blood flow, and protects cardiomyocytes from ischemia and metabolic stress.

Using genetic and pharmacological approaches, the inventors' laboratory have established the indispensable role of alpha-calcitonin gene related peptide (a-CGRP) in a variety of cardiovascular diseases, including experimental hypertension, myocardial infarction, ischemic-reperfusion cardiac injury, and heart failure. α-CGRP, a 37-amino acid regulatory neuropeptide, is generated from the alternative splicing of the primary transcript of the calcitonin/a-CGRP gene, CALC I. α-CGRP synthesis occurs in the central and peripheral nervous systems particularly in the sensory neurons of the dorsal root ganglia which terminate peripherally on blood vessels. α-CGRP signals are mediated via a complex membrane receptor composed of three proteins: (i)- a seven transmembrane G-protein coupled receptor, known as the calcitonin receptor-like receptor (CLR), (ii) a single transmembrane protein- Receptor Activity Modifying Protein (RAMP), and (iii) a small intracellular protein called Receptor Component Protein (RCP). Protein RAMP-1 helps in trafficking of CLR from the endoplasmic-reticulum/Golgi complex to the cell membrane.

Peptide α-CGRP is the most potent vasodilator known and exhibits positive chronotropic and inotropic effects. Systemic administration of α-CGRP decreases blood pressure in normotensive and hypertensive animals and humans. Various animal and cell culture based studies confirm that α-CGRP decreases angiotensin II activity, increases cardiac blood flow, and protects cardiomyocytes from ischemia and metabolic stress.

The inventors' laboratory has also demonstrated that α-CGRP acts as a compensatory depressor to attenuate the rise in blood pressure in three different models of experimental hypertension: 1) deoxycorticosterone (DOC)-salt, 2) subtotal nephrectomy-salt, and 3) L-NAME induced hypertension during pregnancy. A similar compensatory depressor role of α-CGRP has also been shown in chronic hypoxic pulmonary hypertension. Using transverse aortic constriction (TAC) induced pressure-overload heart failure mouse model, the inventors' laboratory demonstrated that TAC pressure-overload significantly enhances cardiac hypertrophy and subsequent cardiac dilation and dysfunction, cardiac apoptosis, fibrosis and inflammation, and mortality in α-CGRP knock-out (KO) mice compared to their counterpart TAC wild-type mice.

These data indicate that α-CGRP is critical to cardio-protection from pressure-overload induced congestive heart failure. The inventors' recent study further confirms the cardio-protective action of native α-CGRP peptide in heart failure using long-term administration of α-CGRP (28 days) in TAC-mice, this administration significantly reduced apoptosis and fibrosis in TAC hearts, and also preserved the hearts at functional and anatomical levels. These studies indicate that α-CGRP is a promising drug candidate to treat cardiovascular diseases. However, the short half-life of α-CGRP in human plasma (t_{1/2} = ~5.5 min) limits the use of α-CGRP as a drug for long-term treatment regimes. Currently, an α-CGRP agonist analogue (an acylated form of α-CGRP with half-life, t_{1/2} = ~7h) has been developed by *Novo Nordisk,* and studies conducted in rodent models of hypertension and heart failure demonstrated that systemic subcutaneous administration of this α-CGRP analogue reversed the renal, vascular, and cardiac damage caused by angiotensin II-induced hypertension or by abdominal aortic constriction (AAC)-induced heart failure.

In recent years, peptoids are gaining considerable attention to modify proteins and peptides in order to increase their stability, efficacy, and physiological properties. Peptoids are peptidomimetic molecules. A peptoid monomer is a *N*-substituted glycine molecule that is structurally identical to α-amino acid except the side chain (R-group) in a peptoid is attached on the nitrogen rather than the α-carbon atom. The side chain (R-group) substitution makes peptoids proteolytically stable while retaining key chemical and physical properties of native amino acid.

The aim of the present disclosure is to protect and highlight the extreme potential of developing a novel peptoid containing α-CGRP analogue, i.e. peptoid-peptide hybrid, with increased stability and efficacy in the human plasma, and its use in cardiovascular diseases. Recently the inventors synthesized a novel α-CGRP agonist analogue containing two peptoid monomers, *N*-methoxyethylglycine (NMEG), at the N-terminal end of human α-CGRP peptide (the inventors designated it as NMEG-αCGRP). The inventors' *in vitro* and *in vivo* experiments show that the synthesized peptoid-peptide hybrid, NMEG-αCGRP, is biologically active and possess no cytotoxicity against cardiac cell lines. These studies suggest that NMEG-αCGRP or any variation of NMEG can be or could be used as a promising therapeutic drug to treat and prevent various cardiovascular diseases, including heart failure, hypertension, myocardial ischemia, cardiomyopathy. and myocardial infarction, etc.

### MATERIAL AND METHODS

### Animals

Eight-week-old C57/BL6 male mice were purchased from Charles River Laboratories (Wilmington, MA), and housed in the institutional animal facility maintained at 25 °C with an automatic 12 h light/dark cycle. All mice were allowed to acclimate for one week before the start of experiments. Mice received a standard diet and tap water *ad libitum.* The animal protocols used for this study were in accordance with the guidelines of the National Institutes of Health (NIH), USA, and were approved by the University of South Carolina Institutional Animal Care and Use Committee.

### Synthesis of NMEG-αCGRP

The N-terminal Rink-amid resin bound full length human α-CGRP (37 amino acids) was synthesized by solid phase Fmoc chemistry at the facility at RS Synthesis (Louisville, KY). The resulting Rink-amide resin bound and F-moc protected human α-CGRP contains disulfide bond at amino acid positions 2 and 7 (Cys2-Cys7), and one -NH₂ group at the C-terminal end. NMEG peptoid was synthesized using solid phase submonomer peptoid synthesis method and two NMEG-moieties were added at the N-terminal end of the synthetic resin bound-aCGRP peptide in the laboratory of Dr. Shannon L. Servoss at University of Arkansas-Department of Chemical Engineering, Fayetteville, Arkansas. F-moc group from amino-acid side chains were removed by standard protocol. The final purity and identity of NMEG-αCGRP was confirmed by analytical reversed-phase high-performance liquid chromatography (RP-HPLC) and electrospray mass spectrometry (MALDI-TOF) at the University of Arkansas mass spectrometry facility.

### Preparation of NMEG-αCGRP stock solution

A stock solution of 0.5 mg/ml of NMEG-αCGRP was prepared in sterile saline solution (0.9% NaCl soln). The prepared solution was filter sterilized with a 0.2 µm syringe filter, and kept at -80 °C until use.

### Measurement of Blood Pressure via Tail cuff

Blood pressure was measured by tail-cuff method using MC4000 Blood Pressure Analysis System (Hatteras Instruments, Cary, NC). Mice were trained at least 3 consecutive days prior to baseline blood pressure measurements to reduce stress-induced changes. Prior to recording blood pressure, mice were normalized in the recording room for at least 1 h, and kept on the instrument platform for 5 min to bring animal body temperature to instrument temperature. After measuring baseline blood pressure (designated as 0 h), NMEG-αCGRP doses (per 25 g mouse) 247.3, 742, 2473, and 7420 picomole in 200 µl sterile saline solution were administered subcutaneously and blood pressure measurements were taken at time points 10 min, 30 min, 1 h, 2 h...up to 3 days. Systolic pressure (mm Hg) was used to prepare drug response curve.

### In vitro cell viability assay

Two cardiac cell lines, rat H9C2 cells and mouse HL-1 cells, were used to determine the toxicity of NMEG-αCGRP in *in vitro* cell culture assays. Rat H9C2 cardiac cell line was maintained in complete culture medium (Dulbecco's Modified Eagle's Medium, DMEM) supplemented with 10% fetal bovine serum (FBS), 100 U/ml penicillin, 100 µg/ml streptomycin and 0.25 µg/ml amphotericin B, and grown at 37 °C in a humidified incubator with 5% CO₂. Mouse HL-1 cardiac muscle cells were maintained in Claycomb Basal Medium (Millipore-Sigma, St. Louis, MO) supplemented with 10% fetal bovine serum (FBS), 0.1 mM norepinephrine in ascorbic acid, 2 mM L-Glutamine, and 1x penicillin/streptomycin soln. HL-1 cells were grown in cell culture plates/flasks coated with gelatin/fibronectin ECM mixture at 37 °C in a humidified incubator with 5% CO₂. Media was exchanges every day.

The viability of cardiac cells in the presence or absence of NMEG-αCGRP was determined by trypan blue cell viability assay. Rat H9C2 cells were grown in presence of different concentrations of NMEG-αCGRP (0.5, 1.5, and 5 µM) in presence of complete culture medium at 37 °C in a humidified incubator with 5% CO₂. Mouse HL-1 cells were treated with NMEG-αCGRP and grown in gelatin/fibronectin coated cell culture plates/flask at 37 °C in a humidified incubator with 5% CO₂. On day 7, both cell lines were photographed under Nikon bright-field inverted microscope (Nikon, Tokyo, Japan). Cells were trypsinized with 0.025% trypsin/EDTA soln and counted using a hemocytometer using the trypan-blue exclusion method (Millipore-Sigma). The experiments were repeated at least three times.

### RESULTS

### Synthesis, Purification, and Characterization of NMEGylated α-CGRP

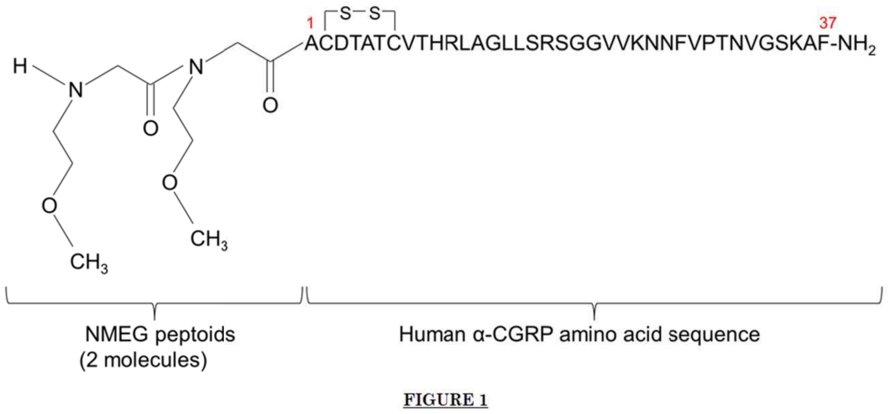

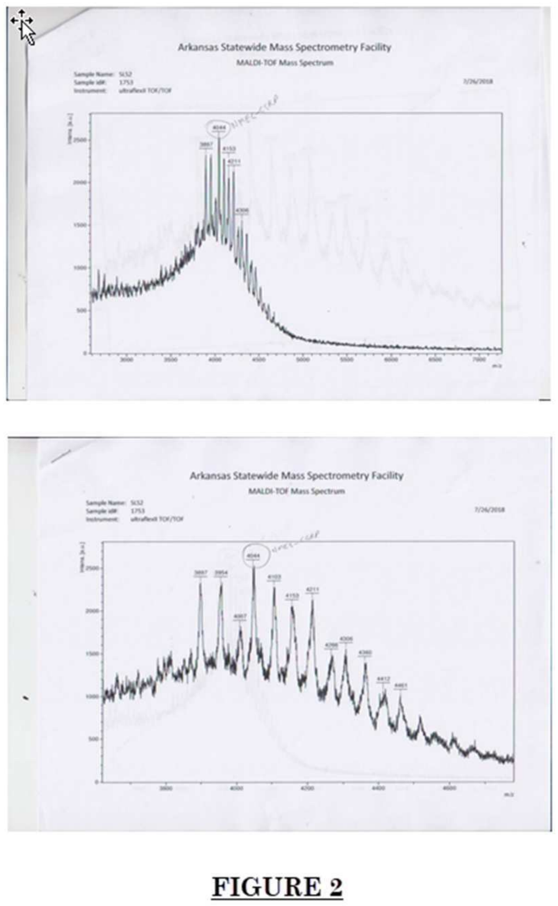

NMEGylated α-CGRP (NMEG-aCGRP) was synthesized by adding two *N*-methoxyethylglycine molecules (NMEG) to the N-terminal end of the α-CGRP peptide using solid phase submonomer peptoid protocol. F-moc protective group was removed from the resin-bound peptide and the peptoid-peptide hybrid was purified by analytical reversed-phase high-performance liquid chromatography (RP-HPLC). The final peptoid-peptide hybrid NMEG-αCGRP contains two molecules of *N-*methoxyethylglycine peptoid at the N-terminus of α-CGRP peptide, see FIG. 1. The identity and molecular mass of the prepared NMEG-αCGRP was determined by electrospray mass spectrometry (MALDI-TOF). The MALDI-TOF analysis showed that the molecular mass of the prepared NMEG-αCGRP was 4044, see FIG. 2 upper and lower panel.

FIG. 1 shows the structure of NMEG-αCGRP. Peptide-peptoid hybrid NMEG-αCGRP contains two molecules of *N*-methoxyethylglycine (NMEG) at the N-terminal end of human α-CGRP peptide. Human α-CGRP contains a disulfide bond (-S-S-) at amino acids 2 and 7 (Cys2-Cys7), and an amine group (-NH₂) at the C-terminal end. First and last amino acid position on α-CGRP was marked as 1 and 37, respectively.

FIG. 2 shows MALDI-TOF analysis of synthesized peptoid-peptide hybrid NMEG-αCGRP. The MALDI-TOF data revealed that the molar mass of NMEG-αCGRP is 4044 (upper and lower panel).

### NMEG-αCGRP reduces blood pressure in wild-type mice

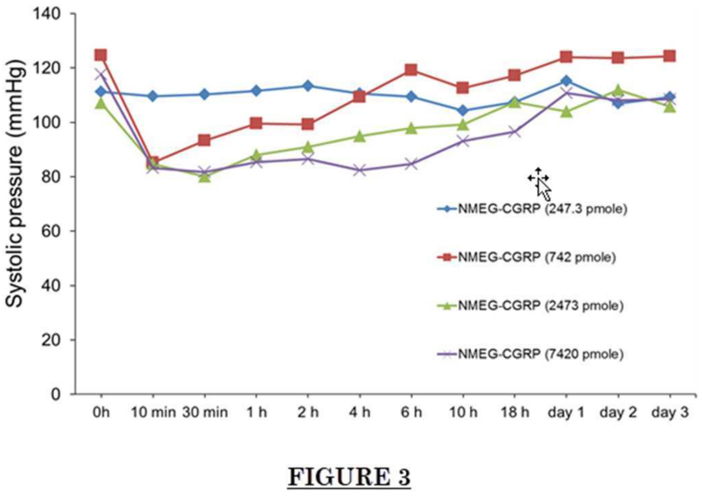

The biological activity of NMEG-αCGRP was determined by measuring its effect on the blood pressure in mice. Different doses of NMEG-αCGRP (247.3, 742, 2473, and 7420 picomole per 25 g mice) were given subcutaneously and blood pressure was measured by tail-cuff method. The administration of 247.3 picomole of NMEG-αCGRP did not significantly change systolic pressure, and remains at baseline. However, starting with 742 picomole of NMEG-αCGRP drastic decrease in blood pressure was observed, see FIG. 3. At these doses, the maximum decrease in blood pressure became apparent at 10 min after the injection, however the time taken to return the blood pressure to baseline is different. Systolic blood pressure returned to almost baseline by 6 h, 18 h, and 24 h with the 742, 2473, and 7420 picomole doses, respectively.

FIG. 3 shows a NMEG-αCGRP dose response curve. The dose response curve showing the effects of subcutaneously administered different concentrations of NMEG-αCGRP on systolic blood pressure (mmHg) in the mice (n= 2 mice per group).

### NMEG-αCGRP did not affect cardiac cell culture viability

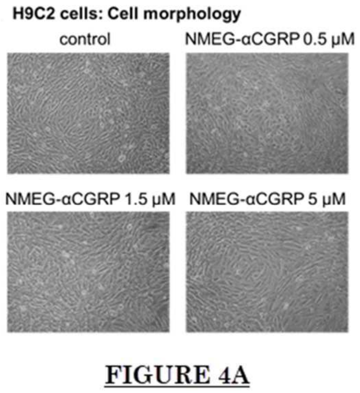

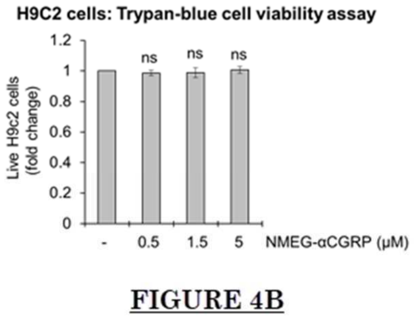

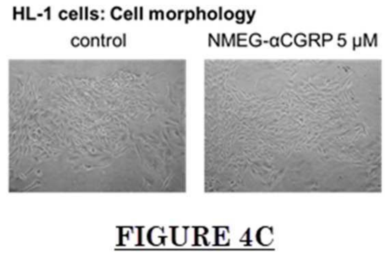

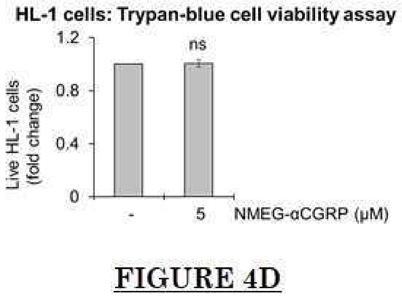

The cytotoxicity of NMEG-αCGRP was confirmed by growing two cardiac cell lines- H9C2 cells and HL-1 cells -in presence of NMEG-αCGRP. After 7 days of treatments, cells were photographed to determine cell morphology and trypan-blue assay were performed to analyze the cell viability. Phase contrast images in FIG. 4 at A demonstrated that the morphology of NMEG-αCGRP treated H9C2 cells was similar to control-nontreated cells. Trypan blue cell-exclusion assay showed that the number of H9C2 live cells was not changed significantly compared to control after 7 days treatment with various concentrations of NMEG-αCGRP, see FIG. 4B. Similarly incubation of 5 µM of NMEG-αCGRP did not affect the morphology and viability of HL-1 cells after 7 days treatment, see FIG. 4 at C and D. These results confirmed that NMEG-αCGRP do not exhibit cellular toxicity against the cardiac cell lines tested.

**FIG. 4** **shows** *in vitro* cell viability assays. FIG. 4A - Representative phase contrast images taken on day 7 after NMEG-αCGRP treatments (0.5, 1.5, and 5 µM) to cardiac H9C2 cells. FIG. 4B - Bar diagrams showing the viability of cardiac H9C2 cells after 7 days treatment with NMEG-αCGRP as determined by trypan blue cell viability assay. Values are average of three experiments and expressed as fold change. *P* value < 0.05 was considered significant. ns= non-significant compared to control. FIG. 4C - Representative phase contrast images of HL-1 cells taken on day 7 after NMEG-αCGRP treatment (5 µM). Cells were trypsinized and live cells were quantitated by trypan blue cell viability assay and plotted as fold change, see FIG. 4D. *P* value < 0.05 was considered significant. ns = non-significant compared to control.

The inventors present study demonstrated that chemically synthesized NMEGylated α-CGRP (NMEG-αCGRP, see FIG. 1, is: (i)- biologically active as subcutaneous administration of NMEG-αCGRP appears to decrease the blood pressure in mice, and (ii) non-toxic to cardiac cells (*in vitro* assays).

In an attempt to develop a stable α-CGRP agonist, the inventors utilized peptoid chemistry to synthesize NMEGylated αCGRP. NMEGylation of αCGRP peptide was carried out by covalently coupling two molecules of *N*-methoxyethylglycine (NMEG) peptoid to the N-terminal end of human full length αCGRP peptide. The amino acid sequence, position of disulfide bond at Cys2 and Cys7 (Cys2-Cys7), and a -NH₂ group at C-terminus end of NMEG-αCGRP is similar to that of native human α-CGRP peptide. The MALDI-TOF analysis revealed that the molecular mass of peptoid-peptide hybrid NMEG-αCGRP is 4044, see FIG. 2, while the native human α-CGRP has molecular weight 3789.33. Thus, a minor modification of the target peptide, i.e. addition of two NMEG monomer to αCGRP, slightly changes its molar mass by 6.7%.

Native α-CGRP is a potent vasodilator and reduces blood pressure in normotensive and hypertensive rodents and humans. The inventors' data in FIG. 3 shows that administration of NMEG-αCGRP lowers the systolic pressure in mice. The hypotensive action of NMEG-αCGRP *in vivo* might be due to the vasodilation, as seen with native αCGRP peptide, where NMEG-αCGRP reduced total peripheral resistance and sustained vasodilation in the vasculature. These data indicate that NMEG-αCGRP possess biological activity as seen with native αCGRP.

N-methoxyethylglycine (NMEG) is a hydrophilic peptoid monomer, and it has been reported that coupling of oligo- or mono-NMEG molecule at either the N- or C-terminus of a peptide C₂₀ greatly improves solubility and serum stability of the C₂₀ peptide. Addition of a linker glycine molecule further enhances the biological activity of the C₂₀ peptide. This study suggests that NMEGylation of peptide is a novel peptoid-based approach to enhance the bioavailability and efficacy of peptides.

Taking advantage of the peptoid chemistry, the inventors synthesized a novel NMEG-αCGRP molecule, see FIG. 1. The inventors will also develop a library of NMEGylated α-CGRPs by adding single or multiple NMEG molecules at different positions on αCGRP peptide, either at the N-terminus, C-terminus, or within the peptide sequence, with or without linker molecule. As peptoids are resistant to proteases, NMEG-αCGRPs can be administered via subcutaneously, intraperitoneally, intravenously, intramuscular, transdermal, topical, intraarterial, intraspinal, intraocular, oral, or through nasal passage.

The prepared NMEG-αCGRP drug formulation can be maintained as a solid, liquid or aerosol form. The possible solid form of drug formulation can be capsules, tablets, pills, powder, creams, solution, elixir, and implantable dosage units in the form of a patch, osmotic pump, or a mechanical device. An implantable dosage unit can be placed on the skin or implanted locally inside the patients' body in places such as the heart, kidney, or artery site. The possible liquid drug formulations can be solution or elixir and adapted for the injection or oral administration of NMEG-αCGRP. Aerosol formulations for NMEG-αCGRP may be in inhaler form for direct delivery to the lungs. The NMEG-αCGRP can be mixed with other matrix/drug-carriers to develop delivery system for timed and controlled release of NMEG-αCGRP. The matrix/drug-carriers can be biocompatible material, in the form of microparticles/nanoparticles using alginate-polymer, including liposomes, exosome, silicone, polyproteins, polyamino acids, polysaccharides, fatty acids, phospholipids, polyglycolide, nucleic acids, polylactic acid, polyesters, polyanhydrides, amino acids, polynucleotides, polyvinylpyrrolidone, polyvinyl propylene, hyaluronic acid, collagen, carboxylic acids, and chondroitin sulfate. Implantable dosage units in the form of a patch, osmotic pump, or a mechanical device may also be used for controlled release of NMEG-αCGRP in the patients' body. The NMEG-αCGRP drug formulation can be administered single or multiple times, given either simultaneously or over an extended period of time, alone or in combination with other drugs and therapies.

**Experimental: encapsulation of α-CGRP in alginate microcapsules and its efficacy in heart failure-mouse model -** The inventors will test the peptoid based technology in various cardiovascular diseases, including myocardial infarction, heart failure, cardiac ischemia, and hypertension induced heart failure and kidney damage, cardiomyopathy induced by chemotherapy (such as doxorubicin) or toxins, hypertension and cardiac remodeling induced during pregnancy. In contrast to peptides, peptoids are easy to synthesize in cost-effective and flexible manner. It makes peptoids and peptoid-containing peptidomimetics ideal tools for drug discovery related studies. Together, the inventors cost-effective peptoid NMEG-based αCGRP modifications will help to develop novel therapeutic agents with increased self-life and enhanced efficacy in human, compared to native αCGRP peptide, and thus benefiting patients suffering from cardiac failure and kidney damage caused by cardiovascular diseases.

**Rationale-** α-CGRP (alpha-calcitonin gene related peptide), a potent vasodilator neuropeptide, has been shown in studies from our laboratory and others to have a protective function in a variety of cardiovascular diseases, including heart failure, myocardial infarction, and experimental hypertension. Our recent study demonstrated that exogenous administration of native α-CGRP using osmotic mini-pumps protected the heart from pressure-induced heart failure in wild-type mice. However, the short half-life of peptide and non-applicability of osmotic pumps in human limits the use of α-CGRP as a therapeutic agent for heart failure.

**Objective-** We sought to comprehensively study a novel α-CGRP delivery system to determine its bioavailability *in vivo* and test the cardioprotective effect and for the first time treatment of alginate-α-CGRP microcapsules in a mouse model of pressure-overload induced heart failure.

**Methods and Results-** Native α-CGRP filled alginate microcapsules (200 micron) were prepared using an electrospray method. Mice were divided into four groups: sham, sham-alginate-α-CGRP, TAC-only, and TAC-alginate-α-CGRP, and transaortic constriction (TAC) procedure was performed in TAC-only and TAC-alginate-α-CGRP groups of mice to induce pressure-overload heart failure. After two-day or fifteen-day post-TAC, alginate-α-CGRP microcapsules (containing 150 µg α-CGRP; final α-CGRP dose 6 mg/kg/mouse) were administered subcutaneously on alternate day, for 28 days, and cardiac functions were evaluated by echocardiography weekly. After 28 days of peptide delivery, all groups of mice were sacrificed, hearts were collected, and biochemical and histological analyses were performed. Our data demonstrated for the first time that administration of alginate-α-CGRP microcapsules significantly improved all cardiac parameters examined in TAC mice. When compared to sham mice, TAC markedly increased heart and lung weight, left ventricle (LV) cardiac cell size, cardiac apoptosis and oxidative stress. In contrast, administration of alginate-α-CGRP microcapsules significantly attenuated the increased heart and lung weight, LV cardiomyocytes size, apoptosis and oxidative stress in TAC mice. Finally, we show that administration of alginate-α-CGRP microcapsules just prior to the onset of symptoms has the ability to reverse the deleterious parameters seen in TAC mice.

Our results demonstrate that encapsulation of α-CGRP in alginate polymer is an effective strategy to improve peptide bioavailability in plasma and increase the duration of the therapeutic effect of the peptide throughout the treatment period. Furthermore, alginate mediated α-CGRP delivery, either prior to onset or after initiation of symptom progression of pressure-overload, improves cardiac functions and protects hearts against pressure-overload induced heart failure.

Alpha-calcitonin gene related peptide (α-CGRP), a 37 amino acid neuropeptide, is considered the most potent vasodilator discovered to date, and possesses positive chronotropic and inotropic effects. Extensive studies from our laboratory and others established a protective function for α-CGRP in a variety of cardiovascular diseases, including heart failure, myocardial infarction, and experimental hypertension. In addition, α-CGRP delivery lowers blood pressure (BP) in normal as well as hypertensive animals and humans. Using α-CGRP knock-out (KO) mice, our laboratory showed that, in comparison with wild-type mice, KO mice exhibited greater cardiac hypertrophy, and cardiac dilation and dysfunction, cardiac fibrosis, and mortality when subjected to transverse aortic constriction (TAC) pressure-overload induced heart failure. Our recent study demonstrated that long-term exogenous delivery of native α-CGRP, through osmotic mini-pumps, attenuated the adverse effects of TAC pressure-overload induced heart failure in wild-type mice. Long term administration of native α-CGRP preserved cardiac function, and reduced apoptotic cell death, fibrosis, and oxidative stress in TAC left ventricles (LVs), thus confirming the cardioprotective function of α-CGRP in congestive heart failure. Similarly, infusion of either native α-CGRP or an α-CGRP-agonist analog (an acylated form of α-CGRP with half-life, t_{1/2}= ~7h) significantly improved cardiac functions in rodent models of hypertension and heart failure. These lines of evidence further confirm that α-CGRP, either native or its derivative, is a promising drug candidate to treat cardiovascular diseases. However, the short half-life of α-CGRP (t_{1/2}= ~5.5 min in human plasma) and non-applicability of implanted osmotic pumps in humans limits the use of α-CGRP as a therapeutic agent for long-term treatment. Therefore, novel delivery systems are needed that could increase the bioavailability of the peptide in the serum.

Alginate polymers have garnered favor recently as a FDA approved novel drug carrier. This is underscored by several clinical trials on alginate-based drug delivery formulations that are currently ongoing. Alginate is a water soluble linear polysaccharide isolated from the brown algae. Structurally, it is an unbranched polyanionic polysaccharides of 1-4 linked α-L-guluronic acid and β-D-mannuronic acid. As the alginate polymer in stable at wide range of temperature (0 - 100 °C), non-toxic, and biocompatible, a variety of biomolecules ranging from peptides, DNA, antibodies, proteins to cells have been used for encapsulation. Our laboratory has routinely utilized alginate-based drug delivery technology to encapsulate various proteins, inhibitors, and cells, to treat both corneal wounds in diabetic rats and macular degeneration in a mouse model.

The aim of the present disclosure was to develop a novel alginate based drug delivery system applicable of long-term sustained release of α-CGRP in humans. We used an electrospray method to encapsulate α-CGRP in alginate microcapsules and tested its efficacy in TAC pressure-overload induced heart failure both as a prevention and treatment. Our results show that subcutaneous administration of alginate-α-CGRP microcapsules immediately after TAC surgery and prior to the onset of symptoms significantly protects hearts at the physiological and cellular level. Thus, our novel state-of-the-art technology to encapsulate α-CGRP and its delivery through alginate microcapsules offers new options to benefit people suffering from cardiovascular diseases.

### METHODS

### Preparation of alginate-α-CGRP microcapsules

An electrospray method was used to prepare α-CGRP encapsulated alginate microcapsules of 200 µm size. Briefly, 2% alginic acid solution (high mannuronic acid content and low viscosity; MilliporeSigma, St. Louis, MO) was prepared in sterile triple distilled water and filtered through 0.2 µm syringe filter. A stock solution of 2 mg/ml of rat/mouse native α-CGRP (GenScript USA Inc., Piscataway, NJ) was prepared in sterile 0.9% NaCl saline solution and further filter sterilized through 2 µm syringe filter. Five hundred microgram of prepared α-CGRP was mixed with 1 ml of 2% alginic acid and passed through positively charged syringe at a constant rate under high voltage current into the 150 mM CaCl₂ gelling solution to make calcium-coated alginate-α-CGRP microcapsules. Prepared microcapsules were washed 4-5 times with sterile triple distilled water for 5 min each to remove excess CaCl₂ and α-CGRP filled microcapsules were finally suspended in 500 µl of sterile triple distilled water. Alginate-only microcapsules were prepared under similar conditions. Release of peptide from alginate-α-CGRP microcapsules was confirmed by *in vitro* α-CGRP release assay. Briefly, 250 µl supernatant was collected at various time points and stored at 4 °C, and the volume was made up each time with sterile water. Peptide concentration in the supernatant was quantitated by MicroBCA protein assay kit (Pierce/ThermoScientific, Waltham, MA) using rat/mouse α-CGRP as standard. Supernatant collected from alginate-only microcapsules was used as control. Final absorbance was measured at 450 nm using Spectramax Plus-384 microplate reader (Molecular Devices, Sunnyvale, CA) and plotted.

### Pressure-overload heart failure mouse model

Eight-week-old male C57/BL6 mice (Charles River Laboratories, Wilmington, MA) were maintained on a 12 h light/12 h dark cycle with free access to standard food and water. Mice were allowed to acclimate for one week after shipment. The animal protocols were approved by the University of South Carolina-Institutional Animal Care and Use Committee following the National Institutes of Health (NIH), USA, guidelines.

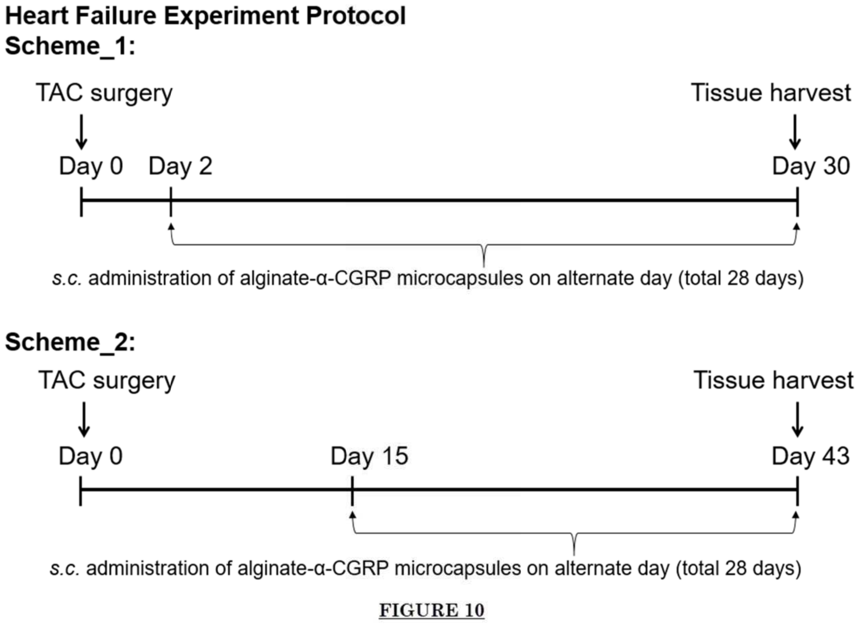

Transverse aortic constriction (TAC) procedure in mice was performed to induce pressure-overload heart failure. Briefly, chest of anesthetized mice (under 1-1.5% isoflurane) was opened through the suprasternal notch, and 7-0 suture (Ethicon prolene polypropylene blue) was passed under the aortic arch between the left common carotid and innominate arteries. The suture was tied around both the aorta and a 27-gauge needle. After placing a knot, the needle was removed. This procedure yield 70-80% aortic constriction. The chest was closed using 6-0 silk suture and mice were allowed to recover. Sham-operated mice underwent an identical procedure except for the aortic constriction. Two days post-surgery, mice were divided into four groups: sham (n= 8), sham-alginate-CGRP (n= 7), TAC-only (n= 7), and TAC-alginate-CGRP (n= 8). In the sham-alginate-CGRP and TAC-alginate-CGRP groups of mice, α-CGRP-encapsulated alginate microcapsules (containing 150 µg of α-CGRP; FINAL α-CGRP DOSE 6 MG/KG/MOUSE) were injected subcutaneously into the flank region of mice on alternate day, for 28 days. At the end of the experiment (day 28 of α-CGRP delivery), mice from all groups were weighed and euthanized. The wet weight of hearts and lungs were measured and photographed. Basal portion of the heart left ventricle (LV) was fixed in 4% paraformaldehyde/PBS (pH 7.4) for histochemistry, while apical portion was snap frozen in liquid N₂ and stored at -80 °C for biochemical analyses. In addition, the treatment protocol was performed for α-CGRP in which mice were divided in to four groups: sham (n= 5), sham-alginate-CGRP (n= 4), TAC-only (n= 4), and TAC-alginate-CGRP (n= 4), and fifteen-day post-TAC, alginate-α-CGRP microcapsules (containing 150 µg of α-CGRP; FINAL α-CGRP DOSE 6 MG/KG/MOUSE) were injected subcutaneously into the flank region of mice on alternate day, for 28 days. The treatment regime for both studies is found in supplemental data, see FIG. 10. At the conclusion of the study (day 28), mice were euthanized, and tissues were collected as discussed before.

### Transthoracic echocardiography

A Vevo 3100 High-Resolution Imaging System (VisualSonics Inc., Toronto, Canada) was used to perform echocardiography in mice. Briefly, mice were sedated under 2% isoflurane and mice heart rate was maintained at 450±20 beats per minute. Short axis B- and M-mode 2D echocardiograms were recorded through the anterior and posterior LV walls at the level of the papillary muscle. Fractional shortening (FS) and ejection fraction (EF) were calculated by the VisualSonics Measurement Software.

### Blood pressure measurement

Blood pressure (BP) of sham and treatment mice was recorded by non-invasive tail-cuff method using MC4000 BP Analysis System (Hatteras Instruments, Cary, NC). To reduce stress-induced changes, mice were trained at least three-to-five consecutive days prior to baseline BP recording. On the day of BP measurement, mice were normalized in the recording room for at least 1 h, and kept on the instrument platform for 5 min to bring animal body temperature to the instrument temperature. After measuring baseline BP (designated as 0 h), alginate microcapsules (with or without α-CGRP) were administered subcutaneously into the flank region of mice and BP was again recorded at various time points.

### Western blotting

Total protein from the LVs was extracted using RIPA cell lysis buffer (Cell Signaling Technology, Danvers, MA), and protein concentration was measured by BCA protein assay kit (Pierce). Equal amount of protein samples (40 pg) were mixed with 5x Laemmli sample buffer, heated at 95 °C for 10 min, and separated on SDS-polyacrylamide gel followed by transfer on PVDF membrane at 100 volt for 3 h in the cold room. Membrane was blocked with 10% non-fat dry milk prepared in TBST (20 mM Tris-Cl, pH 7.4; 150 mM NaCl with 0.1% Tween-20) for 4 h at room temperature and further incubated in primary antibodies for overnight at 4 °C. Protein signals were detected by adding HRP-conjugated secondary antibodies (Bio-Rad Laboratories, Hercules, CA) for 2 h at room temperature and using Clarity Western Detection Kit (Bio-Rad). Primary antibodies used were cleaved caspase-3 and β-actin (Cell Signaling Technology).

### Immunohistochemistry

Paraformaldehyde-fixed paraffin-embedded LV sections (5 µm) were deparaffinized and rehydrated with xylene and graded ethanol (100%, 95%, and 70%), respectively, and boiled in 10 mM sodium citrate buffer (pH 6.0) for 30 min for antigen retrieval. After permeabilization with 0.2% Triton X-100/PBS for 10 min, LV sections were blocked with 10% IgG-free-BSA/PBS (Jackson ImmunoResearch Laboratories, West Grove, PA) and incubated with primary antibodies for overnight at 4 °C. Alexafluor-488 or Alexafluor-546 conjugated secondary antibodies (Invitrogen, Carlsbad, CA) were added to detect protein signals. After mounting with antifade-mounting media (Vector Laboratories, Burlingame, CA), tissue sections were examined under Nikon-E600 fluorescence microscope (Nikon, Japan). Primary antibodies used were: cleaved caspase-3 (Cell Signaling) and anti-4-hydroxy-2-nonenal (4-HNE; Abcam Inc, Cambridge, MA). DAPI (4', 6-diamidino-2-phenylindole; Sigma) was used to stain nuclei.

Hematoxylin and Eosin (H&E) staining, Texas Red-X conjugated wheat germ agglutinin staining (WGA staining; Invitrogen) and Masson's trichrome-collagen staining (PolyScientific, Bay Shore, NY) were performed using vendors' protocol to measure LV cardiac cell size, cardiomyocyte cross-sectional area, and fibrosis, respectively, and quantitated using NIH-ImageJ software (NIH, USA).

### Cardiac cell lines and in vitro cytotoxicity assays

***Trypan-blue cell viability assay:*** The rat cardiac H9C2 cells were grown at 37 °C in a humidified incubator with 5% CO₂ in complete culture medium (containing DMEM supplemented with 10% fetal bovine serum, FBS, 4.5 gm/liter D-glucose, and 1x penicillin/streptomycin). The viability of H9C2 cells in presence of alginate-α-CGRP microcapsules was determined by trypan-blue assay (Sigma). Briefly, stock solution of rat/mouse α-CGRP (1 mg/ml) was prepared in sterile 0.9% NaCl solution and filter sterilized through 0.2 µm syringe filter. H9C2 cells, grown in complete culture medium, were treated with alginate-only, α-CGRP, or alginate-α-CGRP microcapsules. Following treatments, cells were photographed under phase-contrast microscope to examine the cell morphology. After 7 days of treatment, cells were trypsinized and counted by hemocytometer using trypan-blue exclusion method.

***Calcium dye fluorescent based assay:*** The mouse cardiac muscle cell line, HL-1 cells, were grown on gelatin and fibronectin-coated cell culture flasks in Claycomb Basal Medium (Sigma) supplemented with 10% FBS, 0.1 mM norepinephrine in ascorbic acid, 2 mM L-glutamine, and 1x penicillin/streptomycin soln. HL-1 cells were maintained at 37 °C in a humidified incubator with 5% CO₂, and cell culture media was exchanged on every day.

A cell permeant calcium dye fluorescent based assay was performed in gelatin and fibronectin-coated 24-well culture plate to observe the viability (beating phenotype) of HL-1 cells. Briefly, at 100% cell confluency, 500 µl of 5 µM cell permeable calcium indicator dye Fluo-4AM (Invitrogen) in HEPES-buffered Hanks' solution was added in each well followed by incubation at 37 °C for 1 h in a humidified incubator. After incubation, cells were washed in Hanks' solution and 500 µl Hanks' solution was added. Cells were immediately viewed using the EVOS FL auto2 microscope (Invitrogen). Using the 10x objective setting, spontaneous contraction of HL-1 cells was video recorded (considered as 0 hour). A volume of 500 µl Hanks' solution containing 10 µM alginate-α-CGRP microcapsules was added and video recorded at every 10 min for 60 min.

### Enzymatic activity assay

GSH-Glo Glutathione assay kit (Promega) was used to measure total glutathione (GSH) content in the LVs following vendor's instructions. Briefly, 10 mg LV heart tissue was homogenized in 1x PBS containing 2 mM EDTA, centrifuged at 12,000 rpm for 15 min at 4 °C, and supernatant was collected. 50 µl of GSH-Glo Reagent was mixed with 50 µl of tissue extract (10 µg) and incubated for 30 min at RT. Next, 100 µl of luciferin detection reagent was added and incubated for an additional 15 min at RT. The signal was measured using a Turner 20/20 luminometer (Promega).

### Statistical analysis

Comparisons were made among the groups using student t-test and one-way ANOVA followed by Tukey-Kramer ad hoc test (GraphPad software, La Jolla, CA). *p* value < 0.05 was considered significant.

### RESULTS

### Encapsulation of α-CGRP and release from alginate microcapsules

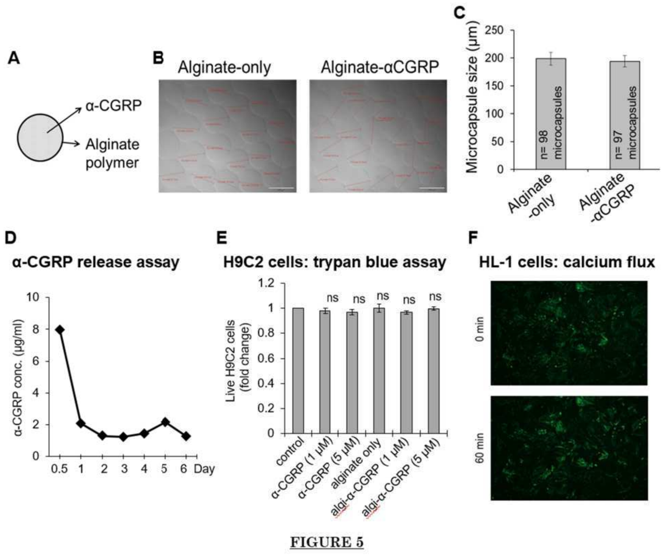

α-CGRP was encapsulated using an electrospray method with following experimental conditions to prepare 200 µm size alginate-α-CGRP microcapsules. α-CGRP (500 µg from a stock 2 mg/ml soln) was mixed with 1 ml of 2% alginic acid solution and loaded to 3 ml syringe attached with high-voltage generator. A beaker filled with 30 ml of ionic gelling bath solution containing 150 mM CaCl₂ was placed below the syringe pump and the distance between the syringe needle to CaCl₂ gelling bath solution was kept 7 mm. As the alginate-α-CGRP mixture was passed through the positively charged syringe needle at a constant rate (flow rate: 60 mm/hr) under high voltage current (6 KV) into the negatively charged CaCl₂ gelling bath, creating spherical Ca⁺²-coated alginate-α-CGRP microcapsules of 200 µm size. We also prepared alginate-only microcapsules of similar size. Prepared microcapsules were photographed and the size of microcapsules was measured. The calculated average size of alginate-only and alginate-α-CGRP microcapsules was 198.84 ± 11.34 µm and 194.23 ± 10.08 µm, respectively (FIG. 5 at A-C). Release of α-CGRP from the prepared alginate-α-CGRP microcapsules was determined by an *in vitro* α-CGRP release assay. FIG. 5 at D showed that presence of α-CGRP was detected in the supernatant for up to 6 days indicating that alginate-α-CGRP microcapsules released peptide over an extended period of time.

### Alginate-α-CGRP microcapsules exhibit no cytotoxicity

It is crucial in determining the effect of the release of α-CGRP on the heart to show that cardiac muscle cells are not altered by the addition of the capsules. To that end we used two different cardiac cell lines- rat H9C2 cells and mouse HL-1 cells, and two different cell viability assays- trypan-blue exclusion assay and calcium dye fluorescent based assay, to determine the cytotoxicity of prepared alginate-α-CGRP microcapsules. H9C2 cells were grown in complete culture medium in presence of 1 µM or 5 µM of alginate-α-CGRP microcapsules. After 7 days of incubation with the capsules, a trypan-blue exclusion assay was carried out. Results from the assay demonstrated that the viability of H9C2 cells was similar among the treatment groups when compared to control-untreated cells (ns= non-significant compared to control, see FIG. 5 at E.

The viability of mouse HL-1 cardiac cells in presence of alginate-α-CGRP microcapsules was determined using an *in vitro* calcium flux fluorescence assay. HL-1 cells stained with Fluo-4AM dye were video recorded to monitor both the beating phenotype and calcium fluxes inside the cell and imaged using an EVOS auto-F2 microscope. After taking images at basal time point (0 min), alginate-α-CGRP microcapsules (10 µM) were added and were further video recorded. Images, see FIG. 5 at F) taken at time points 0 min and 60 min after addition of alginate-α-CGRP microcapsules demonstrated that the alginate-α-CGRP microcapsules (10 µM) did not affect the myocyte contraction of HL-1 cells. These data support our statement that alginate-α-CGRP microcapsules do not exhibit cytotoxicity against the cardiac cell lines tested.

### Alginate-α-CGRP microcapsules reduces blood pressure in mice

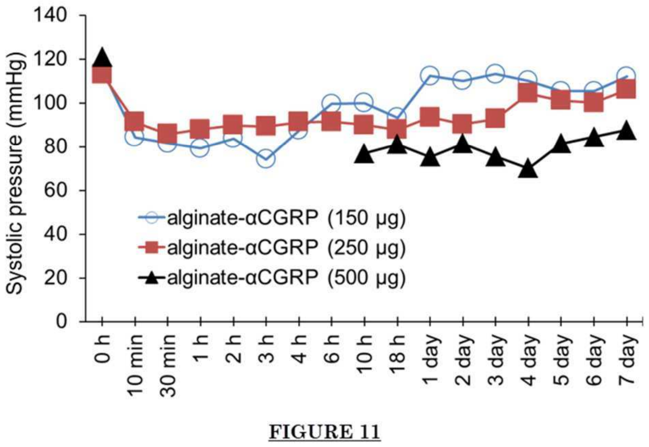

α-CGRP is well-known to reduce BP, thus we set out to confirm the biological activity of released α-CGRP from alginate-α-CGRP microcapsules by measuring changes in BP. Three different doses of alginate microcapsules containing 150 µg, 250 µg, or 500 µg α-CGRP were injected subcutaneously in mice (2 mice/dose) and systolic pressure was monitored at various time points. Data shown in FIG. 2 demonstrates that 150 µg and 250 µg alginate-α-CGRP microcapsules lowered the systolic pressure for up to 18 h and 3 days, respectively, after which time the BP returned to basal level. Subcutaneous administration of 500 µg alginate-α-CGRP per 25 g mouse microcapsules drastically reduced the BP for first 6 h so much so that it could not be recognized by the instrument but by 10 h it registered low and remained below basal levels over the subsequent 7 days. However, subcutaneous administration of an equal amount of alginate-only microcapsules had no effect on the BP in mice (data not shown). These data confirm that α-CGRP is being released from the alginate microcapsules under *in vivo* conditions and that the released α-CGRP remains biologically active for an extended period of time. FIG. 11 shows blood pressure measurements of mice with alginate-α-CGRP microcapsules.

### Alginate-α-CGRP microcapsules delivery improves cardiac functions in TAC mice

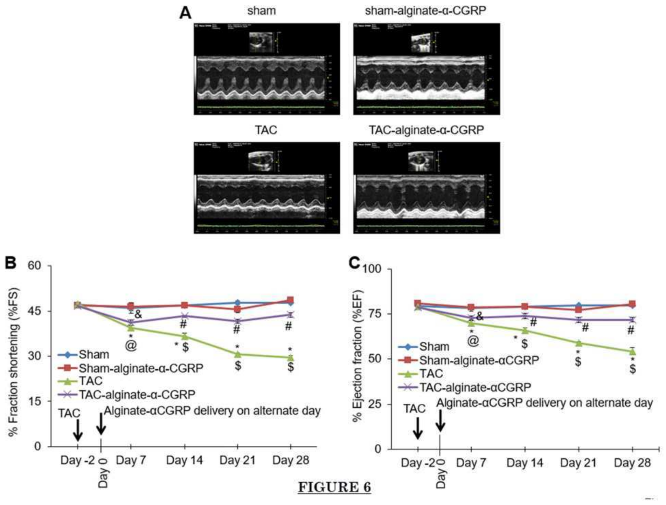

Our previous studies demonstrated that continual α-CGRP administration following TAC surgery showed a cardioprotective capability. Therefore to determine if the alginate-α-CGRP microcapsules also had a cardioprotective effect, B- and M-mode 2D electrocardiography was performed on every 7^{th} day, up to day 28, following subcutaneous administration of 150 µg alginate-α-CGRP microcapsules (final α-CGRP dose 6 mg/kg/mouse), FIG. 6 at A-C. Over the course of experiment, LV systolic function was assessed by measuring both % fraction shortening, see FIG. 6 at B, and ejection fraction, see FIG. 6 at C. Both measures were significantly decreased as expected in the TAC mice when compared to the sham mice. However, repeated administration of alginate-α-CGRP microcapsules starting 2 days after TAC surgery showed significant preservation of both cardiac parameters in treated TAC mice.

### α-CGRP administration attenuates cardiac hypertrophy and fibrosis in TAC mice

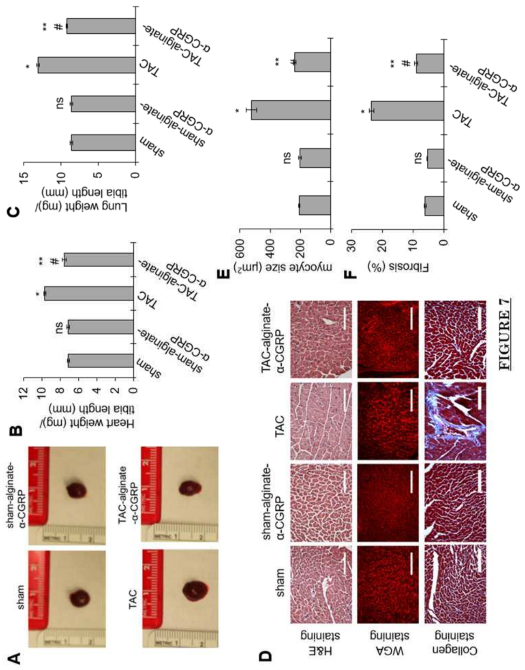

In order to determine if the cardiac cellular damage was also attenuated by alginate-α-CGRP microcapsule treatment, gross and histological measurements were taken of hearts from all of the groups. At the conclusion of the experiment, all groups, treated and sham, were sacrificed. Hearts and lungs were isolated, photographed, and the ratio of wet heart weight to tibia length and wet lung weight to tibia length were measured as indices of LV hypertrophy and dilation and pulmonary congestion, see FIG. 7 at A-C. The representative photographs and bar diagrams in FIG. 7 at A and B show that hearts from TAC mice were larger than that from the sham mice (**p* < 0.05, TAC-only vs sham). Additionally, hearts from mice treated with alginate-α-CGRP microcapsules was significantly smaller than TAC (***p* < 0.05, TAC-alginate-α-CGRP vs TAC) and comparable to sham hearts (#*p >* 0.05, TAC-alginate-α-CGRP vs sham-only; FIG. 7 at A and B). Similarly, the calculated mean lung weight/tibia length was significantly greater in TAC mice compared to sham mice (**p* < 0.05, TAC vs sham) while the increase in lung weight/tibia length after TAC was significantly reduced by α-CGRP administration (***p* < 0.05, TAC-alginate-α-CGRP vs TAC-only, see FIG. 7 at C). The lung weight between TAC-alginate-α-CGRP and sham group of mice was not significantly different (#*p* > 0.05, TAC-alginate-α-CGRP vs sham). The heart size and the ratios heart weight/tibia length and lung weight/tibia length among the sham-alginate-α-CGRP mice and sham-only mice appeared nearly identical (ns, sham-alginate-α-CGRP vs sham-only; FIG. 7 at A-C).

To determine the effect of alginate-α-CGRP microcapsule treatment on cardiac myocyte size, H&E staining and wheat germ agglutinin (WGA) staining was performed, see FIG. 7 at D. As expected, the TAC procedure markedly increased myocytes size in the LVs (**p* < 0.05, TAC vs sham, see FIG. 7 at E). However, LV myocytes size in the TAC-alginate-α-CGRP group was significantly decreased compared to TAC-only mice and was almost identical to sham-only mice (***p* < 0.05, TAC-alginate-α-CGRP vs TAC-only; and #*p* > 0.05, TAC-alginate-α-CGRP vs sham). Treatment with alginate-α-CGRP microcapsules did not affect LV cardiomyocyte size in sham-alginate-α-CGRP mice when compared to sham LV (ns= nonsignificant vs sham). Likewise, when compared to sham, TAC surgery significantly increased LV fibrosis which was decreased with α-CGRP administration in TAC mice (**p* < 0.05, TAC vs sham; ***p* < 0.05, TAC-alginate-α-CGRP vs TAC; #p < 0.05, TAC-alginate-α-CGRP vs sham, see FIG. 7 at D and F).

### α-CGRP administration reduces apoptosis and oxidative stress in TAC LVs

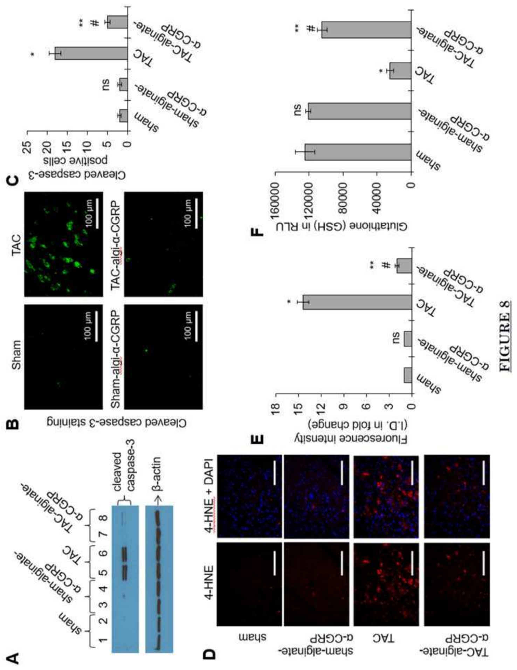

Following TAC, there is an increase in cell death and an elevation in oxidative stress markers. We therefore set out to determine if α-CGRP administration could mitigate these responses. Western blot analysis for the presence of apoptosis markers demonstrated that cleaved caspsase-3 (a marker of apoptotic cell death) was significantly higher in TAC LVs compared to sham LV, and alginate-α-CGRP microcapsules administration significantly reduced cleaved caspsase-3 levels to those observed in sham LVs, see FIG. 8 at A. Similarly, the number of cleaved caspase-3 positive cells (green) were higher in TAC LVs when compared to the sham LV (**p* < 0.05, TAC vs sham, FIG. 8 at B and C). Similarly, when we analyzed the number of cleaved caspase-3 positive cells we determined that it was significantly lower in the TAC-alginate-α-CGRP LVs to TAC LVs and comparable to that of sham LVs (***p* < 0.05, TAC-alginate-α-CGRP vs TAC; #p < 0.05, TAC-alginate-α-CGRP vs sham; FIG. 8 at B and C).

We also examined the hearts for 4-HNE, a marker of oxidative stress-induced lipid-peroxidation. Sections of LVs were images and its immunofluorescence quantitated. We observed that TAC induced pressure-overload markedly increased formation of HNE-adduct in TAC-LV (**p* < 0.05, TAC vs sham; FIG. 8 at D-E), and α-CGRP administration significantly reduced the intensity of signal of 4-HNE in the TAC LV and was comparable to their sham counterpart (***p* < 0.05, TAC-alginate-α-CGRP vs TAC; #*p* < 0.05, TAC-alginate-α-CGRP vs sham). FIG. 8 at F showed that the total glutathione level was significantly reduced in the TAC LVs (**p* < 0.05, TAC vs sham) while significantly restored by treatment of alginate-α-CGRP microcapsules (***p* < 0.05, TAC-alginate-α-CGRP vs TAC; #*p* < 0.05, TAC-alginate-α-CGRP vs sham). All of the oxidative stress parameters in sham-alginate-α-CGRP LVs were comparable with sham LVs (ns= non-significant compared to sham; FIG. 8 at D-F). These results suggest that α-CGRP delivery through alginate microcapsules protected cardiac cells from pressure-overload induced apoptosis and oxidative stress.

### Alginate-α-CGRP microcapsules administration improves cardiac function in 15-day post TAC-mice

Our results from these experiments demonstrated that α-CGRP microcapsule delivery, beginning two-day post-TAC, protected mice against adverse pressure-induced cardiac effects. We next wanted to determine if our alginate-α-CGRP microcapsules could ameliorate these effects after the progression of heart failure had already begun. This would move our studies from a preventive approach to an actual treatment approach. To address this, we again performed TAC surgery in mice, and then 15 days after TAC, alginate-α-CGRP microcapsules (containing 150 pg α-CGRP; final α-CGRP dose 6 mg/kg/mouse) were administered s.c. on alternate days for an additional 28 days. Day 15 was chosen as it's a timepoint when all deleterious measures of heart failure are present in mice following TAC surgery. Echocardiogram data showed the usual result that TAC significantly reduced cardiac fraction shortening (FS) (**p* < 0.05, TAC vs sham). What was exciting was that alginate-α-CGRP microcapsules administration attenuated the reduction in FS following 28 days of treatment. The FS in TAC-alginate-α-CGRP mice was significantly improved compared to TAC mice and was comparable with that of sham mice ($*p* < 0.05, TAC vs TAC-alginate-α-CGRP at the same time point), see FIG. 9 at A. When compared to TAC mice, the wet heart wt and lung wt in TAC-alginate-α-CGRP mice was significantly lower indicating that α-CGRP delivery significantly inhibited cardiac hypertrophy and pulmonary edema in TAC-mice, see FIG. 9 at B-D. During the length of experiment, the TAC group of mice gained only 2% body wt. while sham, sham-alginate-α-CGRP, and TAC-alginate-α-CGRP group of mice gained (in %) 11, 10, and 7 body wt, respectively, indicating that α-CGRP improved body gain in TAC mice, see FIG. 9 at E. Moreover, administration of alginate-α-CGRP microcapsules starting at day 15, significantly attenuated the increased size of cardiomyocytes, see FIG. 9 at F and G, and fibrosis (as determined by collagen content after Masson's trichrome collagen staining; FIG. 9 at F and H) in TAC-LVs after 28 days of treatment. Although α-CGRP concentration used in present study significantly inhibited fibrosis in TAC-LVs, it did not reduce the level to that observed in sham-LVs, see FIG. 9 at H. Our CGRP-treatment study demonstrated, for the first time, that α-CGRP alginate microcapsules administration beginning 15-days post-TAC protected hearts both at physiological and pathological levels and reversed the deleterious effects of pressure overload in heart.

Using genetic and pharmacological approaches, a series of independent studies from our laboratory established that α-CGRP deletion makes the heart more vulnerable to heart failure, hypertension, myocardial infarction, and cardiac and cerebral ischemia indicating α-CGRP is protective against various cardiac diseases. Hearts from the α-CGRP KO mice exhibited a significant reduction in cardiac performance following I/R injury due to elevated oxidative stress and cell death when compared with their WT counterparts. A similar cardioprotective role of α-CGRP has been determined in murine models of hypertension including deoxycorticosterone (DOC)-salt, subtotal nephrectomy-salt, L-NAME-induced hypertension during pregnancy, a two-kidney one-clip model of hypertension, and in chronic hypoxic pulmonary hypertension. Moreover, several human and animal studies showed that exogenous delivery of α-CGRP peptide benefits against cardiac diseases. In patients with stable angina pectoris, intracoronary infusion of α-CGRP delayed the onset of myocardial ischemia. Also, in patients with congestive heart failure, an acute intravenous infusion of α-CGRP improves myocardial contractility and thus improving cardiac functions. Similarly, infusion of α-CGRP in patients with heart failure decreased systemic arterial pressure. Our previous study confirmed that long-term administration of native α-CGRP, through osmotic mini-pumps, significantly preserve the hearts at functional and anatomical levels in TAC pressure-overload mice. A similar study using α-CGRP KO mice presented data that supports our findings on the cardioprotective role of α-CGRP in cardiac diseases and showed that native α-CGRP delivery through osmotic mini-pumps corrected adverse effects of hypertension in these KO mice. Furthermore, subcutaneous administration of an acylated version of α-CGRP, a stable α-CGRP agonist, significantly reduced cardiac hypertrophy, fibrosis, inflammation and oxidative stress in rodent models of hypertension and heart failure. Together, these studies establish α-CGRP as a promising drug candidate to treat and prevent cardiovascular diseases. However, the low bioavailability of the native peptide in human plasma (t_{1/2}= ~5.5 min) makes it difficult to use α-CGRP as a therapeutic agent in a long term treatment regime. Moreover, the applicability of osmotic mini-pump as a peptide delivery system is not feasible in humans. In light of this, new approaches are warranted if α-CGRP is to be an effective and accessible treatment for heart failure.

The present study demonstrated that using an alginate polymer as a drug carrier for α-CGRP was effective in ameliorating pressure-overload induced heart failure. Moreover, cell apoptosis and oxidative stress that accompanies worsening heart failure was reduced by the treatment with alginate-α-CGRP microcapsules. Several lines of evidence demonstrated that systemic administration of α-CGRP reduces BP, however, the reduction in blood pressure is very short because the half-life of native α-CGRP in human plasma is only 5.5 min. We previously used alginate microencapsulation to treat numerous ocular and skin wounds. Recently we used cellular alginate microencapsulation to treat and improve the symptoms of macular degeneration in a mouse model. Alginate is a natural polysaccharide extracted from seaweeds and has been extensively used to encapsulate a wide range of molecules-ranging from large macromolecules, such as cells, DNA and protein, to small molecules- peptides and antibodies. In the current study we developed a novel alginate based α-CGRP delivery system to deliver α-CGRP in controlled and sustained manner. Our state-of-art technology used an electrospray method to prepare α-CGRP encapsulated alginate microcapsules of a consistent size and release. The advantage of using an electrospray method is that the alginate-α-CGRP capsules can range from nano-to micro-size (ranging from 10 nm-500 µm) by adjusting the experimental parameters, e.g., the voltage, flow rate, and distance between needle to gelling bath solution. In addition, one can modify the microcapsule to release its contents at the desired interval.

Encapsulated microcapsules are very stable at room temperature as the spherical shape of alginate-alone and alginate-α-CGRP microcapsules in deionized water was remained intact even after 15 months (data not shown). Encapsulated peptide remained biologically active in vivo as released α-CGRP from subcutaneously administered alginate-α-CGRP microcapsules lowered the BP, an inherent property of native α-CGRP, in mice, see FIG. 11. Also, alginate-α-CGRP microcapsule formulation is non-toxic to cardiac cells, see FIG. 5 at E and F. Alginate-α-CGRP microcapsules up to 5 µM (maximum concentration tested) did not affect the growth of H9C2 cells, see FIG. 5 at E. Similarly, HL-1 cells kept beating on the plate even after 1 h incubation with 10 µM alginate-α-CGRP microcapsules, see FIG. 5 at F. These data indicated that alginate-α-CGRP microcapsules neither affect viability nor beating phenotype of cardiac cells under *in vitro* conditions.

Another important finding of the study is that alginate-α-CGRP microcapsules (containing 150 µg α-CGRP; final α-CGRP dose 6 mg/kg/mouse) subcutaneously administered in pressure-overload heart failure mice, improved myocardial function by restoring both FS and EF, hallmarks of increasing heart failure and attenuated increased apoptotic cell death and oxidative stress in TAC-LVs.

Previously, it has been shown that intravenous injections of α-CGRP significantly decreases mean arterial pressure (MAP) in a dose-dependent fashion in both normal and spontaneously hypertensive rats, however, MAP returns to normal baseline after 20 min of injection in both groups of rats. Our findings demonstrated that subcutaneous administration of 150 µg and 250 µg of alginate-α-CGRP microcapsules (per 25 g mouse) lowered the systolic pressure for 18 h and 3 days, respectively. Moreover, our results indicate that addition of alginate-α-CGRP microcapsules extends the release of peptide, and released α-CGRP remains biologically active for extended periods of time.

Another novel and exciting finding of the present study is that when alginate microcapsules were administered starting at 15-day post-TAC mice there was an immediate reversal of symptoms. This was similar to the ability of α-CGRP filled alginate microcapsules to significantly protect hearts when administered immediately after surgery. Also similar to early administration, treatment started at 15 days post TAC was able to reverse all of the parameters of heart failure examined to include, cardiac hypertrophy, apoptosis, cardiac function and fibrosis. This is the first demonstration that addition of α-CGRP just prior to the onset of symptoms could reverse quickly the damage that is observed with TAC induced heart failure.

Alginate is non-toxic and immunologically inactive, hence prepared alginate based drug formulation does not exhibit side effects and has been FDA approved for use in humans. Our laboratory has established that alginate microcapsules can also undergo freeze-thaw cycles as well as can be lyophilized without compromising the integrity of microcapsules (Data not shown). The lyophilized form of alginate microcapsules immediately swell and regain their shape when suspended in distilled water. Consequently, alginate-α-CGRP microcapsules can be stored at very low temperature and lyophilized to make their easy transport. With these advantages, alginate-α-CGRP microcapsules can be employed as an effective way for controlled and sustained delivery of α-CGRP in humans suffering from cardiovascular diseases. The success of this novel drug delivery technology will have the potential to dramatically change conventional drug therapies used presently to treat the failing heart.

All together these data indicate that an alginate microcapsules based delivery system is an effective strategy to improve α-CGRP bioavailability in plasma and, thus, increase the duration of the therapeutic effect of the peptide throughout the treatment period. In addition, the observed cardioprotective effects of alginate-α-CGRP microcapsules was present either administering prior to symptoms (i.e.. CGRP-prevention study) or at 15 days post-TAC when symptoms are beginning (i.e., CGRP-treatment study). Thus, our study suggests that the developed alginate-α-CGRP microcapsule administration can be effective in the prevention and represents a new treatment of heart failure.

### FIGURE LEGENDS

FIG. 5 at (A-C) - Electrospray method was used to encapsulate α-CGRP in alginate polymer. Prepared alginate-only and alginate-α-CGRP microcapsules were photographed **(B)** and size was measured and plotted **(C). (D)-** An *in vitro* α-CGRP release assay showing amount of α-CGRP released in supernatant from alginate-α-CGRP microcapsules. **(E)-** Bar diagram showing number of live H9C2 cells, as measured by trypan-blue cell viability assay, after 7 days incubation with different concentration of α-CGRP-alone, empty-alginate microcapsules, and alginate-α-CGRP microcapsules. ns= not significant compared to control. **(F)-** The viability of mouse HL-1 cardiac cells in presence of alginate-α-CGRP microcapsules (10 µM) was determined by *in vitro* calcium flux fluorescence assay as discussed in methods section. HL-1 cells stained with Fluo-4AM dye were imaged using EVOS auto-F2 microscope at 0 min and 60 min after addition of alginate-α-CGRP microcapsules (10 µM).
FIG. 4 - Graph showing the systolic pressure, as measured by tail-cuff blood pressure method, after subcutaneous injection of various concentrations of alginate-α-CGRP microcapsules in mice.
FIG. 6 at A - Representative echocardiograms showing short axis B- and M-mode 2D echocardiography performed after 28 days delivery of alginate-α-CGRP microcapsules in sham and TAC-mice. Percentage fractional shortening (FS) and ejection fraction (EF) was calculated at various time points and plotted (B and C).
FIG. 7 at A - Representative images showing the size of the hearts after 28 days delivery of alginate-α-CGRP microcapsules. (B and C)- Bar diagrams showing the ratio of wet heart weight/tibia length, and wet lung weight/tibia length. (D)- The paraffin-embedded LV sections were stained with H&E, WGA stain, and Trichrome-collagen stain. Scale bar= 100 µm. WGA stained sections were used to measure cardiomyocyte size in LVs by NIH-ImageJ software and plotted **(E).** LV collagen content, an indicator of fibrosis, was quantitated by NIH-ImageJ software and plotted **(F).** Values were expressed as the mean ± SEM. **p* < 0.05, TAC vs sham; ***p* < 0.05, TAC-alginate-α-CGRP vs TAC; #*p* > 0.05, TAC-alginate-α-CGRP vs sham; ns= non-significant compared to sham.
FIG. 8 at A - Western blot showing level of cleaved caspase-3 protein in LVs from sham, sham-alginate-α-CGRP, TAC, and TAC-alginate-α-CGRP. β-actin was used as control. (B)- Representative fluorescence images showing cleaved caspase-3 staining (green) to detect apoptosis in the LV sections. Scale= 100 µm. Cleaved caspase-3 positive cells (green) were counted and plotted as the mean ± SEM (C). (D and E)- Fluorescence images showing 4-HNE staining (a marker of lipid peroxidation) in the paraffin-embedded LV sections. DAPI was used to stain nuclei. Scale= 100 µm. The fluorescence intensity of 4-HNE (red) was quantitated by NIH-ImageJ software and plotted as the mean ± SEM. I.D.= integrated density. (F)- Bar diagrams showing glutathione (GSH) level in the LVs. Values were expressed as the mean ± SEM and *p* < 0.05 was considered significant. **p* < 0.05, TAC vs sham; ***p* < 0.05, TAC-alginate-α-CGRP vs TAC; #*p* > 0.05, TAC-alginate-α-CGRP vs sham; ns= not-significant compared to sham.
FIG. 9 at A - Graph showing %FS in sham, sham-alginate-α-CGRP, TAC-only, and TAC-alginate-α-CGRP groups of mice. After 15 days of TAC, alginate-α-CGRP microcapsules (α-CGRP dose 6 mg/kg/mouse) were injected on alternate day, till day 28. Echocardiography was performed at different time points and % FS was plotted as mean ± SEM. **p* < 0.05, TAC vs sham at the same time point; #p < 0.05, TAC-alginate-α-CGRP vs sham at the same time point; $*p* < 0.05, TAC vs TAC-alginate-α-CGRP at the same time point. (B). Representative images showing the size of hearts after 28 days delivery of alginate-α-CGRP microcapsules. Ratio of wet heart weight/tibia length was plotted as mean ± SEM (C). (D)- Bar diagram showing ratio of wet lung weight/tibia length as mean ± SEM. (E)- Bar diagram showing mice weight gain (in percentage) during the course of experiment as mean ± SEM. *p* < 0.05 was considered significant. **p* < 0.05, TAC vs sham; ***p* < 0.05, TAC-alginate-α-CGRP vs TAC; *#p* > 0.05, TAC-alginate-α-CGRP vs sham; ^{@}*p* < 0.05, TAC-alginate-α-CGRP vs sham; ns= not- significant compared to sham. (F)- Representative histology images showing size of cardiomyocytes (WGA staining) and level of fibrosis (trichrome-collagen staining) in the LVs from different groups of mice. Cardiomyocyte size (G) and % fibrosis (H) in LVs was quantitated using NIH-ImageJ software and plotted as mean ± SEM. *p* value < 0.05 was considered significant. **p* < 0.05, TAC vs sham; ***p* < 0.05, TAC-alginate-α-CGRP vs TAC; #*p* > 0.05, TAC-alginate-α-CGRP vs sham; ^{@}*p* < 0.05, TAC- alginate-α-CGRP vs sham; ns= not-significant compared to sham.

### Amino Acid Sequences

A)- Peptide human α-CGRP amino acid sequence- 
S—S
1                                                                                    37
ACDTATCVTHRLAGLLSRSGGVVKNNFVPTNVGSKAF-NH2
Sequence Listing Free Text 
Ala - Cys - Asp - Thr - Ala - Thr - Cys - Val - Thr - His - Arg - Leu - Ala - Gly - Leu - Leu - Ser - Arg - Ser - Gly
- Gly - Val - Val - Lys - Asn - Asn - Phe - Val - Pro - Thr - Asn - Val - Gly - Ser - Lys - Ala - Phe
B)- Peptide rodent (mouse or rat) α-CGRP amino acid sequence- 
S—S
1                                                                                    37
SCNTATCVTHRLAGLLSRSGGVVKDNFVPTNVGSEAF-NH2
Sequence Listing Free Text 
Ser - Cys - Asn - Thr - Ala - Thr - Cys - Val - Thr - His - Arg - Leu - Ala - Gly - Leu - Leu - Ser - Arg
- Ser - Gly - Gly - Val - Val - Lys - Asp - Asn - Phe - Val - Pro - Thr - Asn - Val - Gly - Ser - Glu - Ala -
Phe
(C)- Derivatives of NMEG-αCGRP peptoid-peptide hybrids-
(C.1)- NMEG-αCGRP peptoid-peptide hybrid-
C.1.a)- (NMEG)n-human αCGRP
C.1.b)- 
1
S—S
37
ACDTATCVTHRLAGLLSRSGGVVKNNFVPTNVGSKAF-NH2
(NMEG)n
 n= one or more than one NMEG peptoid monomer

Addition of NMEG-peptoid (alone or in combination) can be at any amino acid on αCGRP sequence (C.1.a). (Here addition of NMEG-molecule at first amino acid on human αCGRP sequence is shown in C.1.b)

### (C.2)- NMEG-αCGRP peptoid-peptide hybrid (with linker molecule):

C.2.a)- (NMEG)n-Linker molecule-human αCGRP
C.2.b)- 
S—S
1                                                                                    37
ACDTATCVTHRLAGLLSRSGGVVKNNFVPTNVGSKAF-NH2
Linker
molecule
(NMEG)n
   n= one or more than one NMEG peptoid monomer
   Linker molecule= glycine, lysine, serine or any other amino acid, or any fatty acid molecule including albumin and casein

Addition of NMEG-Linker molecule (alone or in combination) can be at any amino acid on αCGRP sequence (C.2.a). (Here addition of NMEG-Linker molecule at first amino acid on human αCGRP sequence is shown in C.2.b)
(C.3)- NMEG-αCGRP peptoid-peptide hybrid (with pseudo-/modified-amino acid)-
C.3.a)- (NMEG peptoid)n-Linker molecule-human αCGRP with pseudo-/modified-amino acid(s)
C.3.b)- 
S—S
1 37
A(pseudo)CDTATCVTHRLAGLLSRSGGVVKNNFVPTNVGSKAF-NH2
with or without
linker molecule
(NMEG)n
   n= one or more than one NMEG peptoid monomer
   Linker molecule= glycine, lysine, serine or any other amino acid, or any fatty acid molecule including albumin and casein

Sequence Legend: Human α-CGRP amino acid sequence (A) and rodent (mouse or rat) α-CGRP (B) have an identical amino acid sequence except at four amino acid positions- 1, 3, 25, and 35. However both, human and rodent (mouse or rat) α-CGRPs, share identical biological activities. Human α-CGRP (A) and rodent α-CGRP (B) are a single peptide of 37-amino acids containing one disulfide bond (-S-S-) between amino acids 2 and 7 (cys2-cys7) and one amide molecule (-NH2) at the C-terminal end. Positions of the first and last amino acid in each peptide sequence is marked as 1 and 37, respectively.
(C)- Derivatives of human αCGRP analogues containing NMEG-peptoid.
(C.1)- NMEG-αCGRP peptoid-peptide hybrid. NMEG-αCGRP peptoid-peptide hybrid can be chemically synthesized by adding one or more than one monomer of NMEG peptoid to any amino acid of αCGRP (C.1.a). A NMEG-αCGRP peptoid-peptide hybrid containing NMEG-molecule at first amino acid of human αCGRP is shown as an example. n= one or more than one NMEG peptoid monomer (C.1.b).
(C.2)- NMEG-αCGRP peptoid-peptide hybrid with linker molecule. NMEG-αCGRP peptoid-peptide hybrid may also be chemically synthesized by adding a linker molecule (glycine, lysine, serine or any other amino acid, or any fatty acid molecule including albumin and casein) between NMEG-peptoid and αCGRP peptide sequence (C.2.a). Addition of NMEG-Linker molecule (alone or in combination) can be at any amino acid on αCGRP sequence. As an example, a NMEG-Linker-αCGRP peptoid-peptide hybrid is shown containing NMEG-Linker molecule at first amino acid of human αCGRP. n= one or more than one NMEG peptoid monomer (C.2.b).
(C.3)- NMEG-αCGRP peptoid-peptide hybrid with pseudo-/modified-amino acid(s). NMEG-αCGRP peptoid-peptide hybrid (with or without linker molecule) will also be chemically synthesized by replacing one or more normal amino acid(s) with pseudo-/modified-amino acid(s) in αCGRP sequence to increase the stability and biological activity of CGRP-analogues (C.3.a). Addition of NMEG-peptoid (with or without linker molecule) can be on normal or pseudo-/modified-amino acid. A NMEG-αCGRP peptoid-peptide hybrid, with or without linker molecule, containing pseudo-alanine in αCGRP sequence is shown as an example (C.3.b).

While the present subject matter has been described in detail with respect to specific exemplary embodiments and methods thereof, it will be appreciated that those skilled in the art, upon attaining an understanding of the foregoing may readily produce alterations to, variations of, and equivalents to such embodiments. Accordingly, the scope of the present disclosure is by way of example rather than by way of limitation, and the subject disclosure does not preclude inclusion of such modifications, variations and/or additions to the present subject matter as would be readily apparent to one of ordinary skill in the art using the teachings disclosed herein.

### CLAUSES

1. A biologically active molecule comprising:
   a peptoid monomer coupled to an end terminus of a peptide; and
   wherein the peptoid monomer coupled to an end terminus of a peptide is encapsulated in an alginate polymer.
2. The biologically active molecule of claim 1, wherein the peptoid monomer comprises N-methoxyethylglycine.
3. The biologically active molecule of claim 1, wherein the peptoid monomer is coupled to an N-terminus end of α-CGRP peptide.
4. The biologically active molecule of claim 1, wherein the peptoid-peptide hybrid is formulated to be administered subcutaneously.
5. The biologically active molecule of claim 1, wherein the alginate polymer comprises an unbranched polyanionic polysaccharides of 1-4 linked α-L-guluronic acid and β-D-mannuronic acid.
6. A method for treating or preventing a cardiovascular disease comprising:
   administering a therapeutically effective amount of a peptoid monomer coupled to an end terminus of a peptide;
   wherein the peptoid monomer coupled to an end terminus of a peptide is encapsulated in an alginate polymer; and
   wherein the peptoid monomer coupled to an end terminus of a peptide is administered prior to onset or after initiation of symptoms.
7. The method of claim 6, wherein the cardiovascular disease is selected from the group consisting of heart failure, myocardial infarction, hypertension, cardiac hypertrophy, coronary artery disease, high blood pressure, stroke, dilated cardiomyopathy, idiopathic dilated cardiomyopathy, inherited cardiomyopathy, diabetic-cardiomyopathy, cardiomyopathy induced by chemotherapy (such as doxorubicin) or toxins, cardiac ischemia, hypertension induced heart failure, or cardiac remodeling induced during pregnancy.
8. The method of claim 6, wherein the peptoid monomer is N-methoxyethylglycine.
9. The method of claim 6, wherein the peptoid monomer is coupled to an N-terminus end of α-CGRP peptide.
10. The method of claim 6, wherein the peptoid monomer coupled to an end terminus of a peptide is administered subcutaneously.
11. The method of claim 6, wherein the alginate polymer comprises an unbranched polyanionic polysaccharides of 1-4 linked α-L-guluronic acid and β-D-mannuronic acid.
12. A novel peptoid-peptide hybrid for use in preventing or treating cardiovascular diseases comprising:
   an α-CGRP agonist analogue containing at least two peptoid monomers at an N-terminal end of a α-CGRP peptide.
13. The novel peptoid-peptide hybrid of claim 12, wherein the at least two peptoid monomers comprise N-methoxyethylglycine.
14. The novel peptoid-peptide hybrid of claim 12, further comprising increased stability in human plasma as compared to α-CGRP.
15. The novel peptoid-peptide hybrid of claim 12, wherein the cardiovascular disease is selected from the group consisting of heart failure, myocardial infarction, hypertension, cardiac hypertrophy, coronary artery disease, high blood pressure, stroke, dilated cardiomyopathy, idiopathic dilated cardiomyopathy, inherited cardiomyopathy, cardiomyopathy induced by chemotherapy (such as doxorubicin) or toxins, diabetic-cardiomyopathy, cardiac ischemia, hypertension induced heart failure, or cardiac remodeling induced during pregnancy.
16. The novel peptoid-peptide hybrid of claim 12, wherein the novel peptoid-peptide hybrid is encapsulated in an alginate polymer.
17. The novel peptoid-peptide hybrid of claim 16, wherein the alginate polymer comprises an unbranched polyanionic polysaccharides of 1-4 linked α-L-guluronic acid and β-D-mannuronic acid.

### SEQUENCE LISTING PART OF THE DESCRIPTION

Organization Applicant
   Street :
   City :
   State :
   Country :
   PostalCode :
   PhoneNumber :
   FaxNumber :
   EmailAddress :
   <110> OrganizationName : UNIVERSITY OF SOUTH CAROLINA
Organization Applicant
   Street :
   City :
   State :
   Country :
   PostalCode :
   PhoneNumber :
   FaxNumber :
   EmailAddress :
   <110> OrganizationName : BOARD OF TRUSTEES OF THE UNIVERSITY OF ARKANSAS
Application Project
   <120> Title : A PEPTOID-PEPTIDE HYBRID, NMEG-aCGRP, AND ITS USE IN
   CARDIOVASCULAR DISEASES
   <130> AppFileReference : 2033101.0000197
   <140> CurrentAppNumber :
   <141> CurrentFilingDate : ____-__-
Earlier Applications
   <150> PriorAppNumber : US 62/880,749
   <151> PriorFilingDate : 2019-07-31
Sequence
   <213> OrganismName : Homo sapiens
   <400> PreSequenceString :
   ACDTATCVTH RLAGLLSRSG GVVKNNFVPT NVGSKAF 37
   <212> Type : PRT
   <211> Length : 37
   SequenceName : 1
   SequenceDescription :
Sequence
   <213> OrganismName : Mus musculus
   <400> PreSequenceString :
   SCNTATCVTH RLAGLLSRSG GVVKDNFVPT NVGSEAF 37
   <212> Type : PRT
   <211> Length : 37
   SequenceName : 2
   SequenceDescription :

## Claims

1. A biologically active molecule comprising:
an α-CGRP agonist analogue comprising at least two peptoid monomers at an N-terminal end of an α-CGRP peptide.

2. The biologically active molecule of claim 1, wherein the α-CGRP agonist analogue comprises a linker between the N-terminal end of an α-CGRP peptide and the two peptoid monomers.

3. The biologically active molecule of claim 2, wherein the linker comprises an amino acid or a fatty acid.

4. The biologically active molecule of claim 3, wherein the linker comprises an amino acid.

5. The biologically active molecule of claim 4, wherein the linker comprises glycine.

6. The biologically active molecule of any of the preceding claims, wherein the peptoid monomer comprises N-methoxyethylglycine.

7. The biologically active molecule of any of the preceding claims, wherein the α-CGRP agonist analogue comprises two peptoid monomers.

8. The biologically active molecule of any of the preceding claims, wherein the biologically active molecule is encapsulated in an alginate polymer.

9. The biologically active molecule of any of the preceding claims, wherein the biologically active molecule is for use in preventing or treating cardiovascular diseases.

10. The biologically active molecule for use according to claim 9, wherein the biologically active molecule is administered subcutaneously.

11. The biologically active molecule for use according to claim 9, wherein the biologically active molecule is administered prior to onset of symptoms.

12. The biologically active molecule for use according to claim 9, wherein the biologically active molecule is administered after the onset of symptoms.

13. The biologically active molecule for use according to claim 9, wherein the cardiovascular disease is heart failure.

14. The biologically active molecule for use according to claim 9, wherein the cardiovascular disease is pressure-overload induced heart failure.

15. A novel peptoid-peptide hybrid for use in preventing or treating cardiovascular diseases comprising:
an α-CGRP agonist analogue comprising at least two peptoid monomers at an N-terminal end of an α-CGRP peptide.
